# EUROPEAN PATENT APPLICATION

(11) **EP 4 085 835 A1**
(43) Date of publication of application: **09.11.2022**
(21) Application number: 21171759.0
(22) Date of filing: 03.05.2021
(51) Int. Cl.: A61B 5/12, A61B 5/11, A61B 5/00, G06F 3/01

(54) **DISCREET HANDS- AND EYES-FREE INPUT BY VOLUNTARY TENSOR TYMPANI MUSCLE CONTRACTION**

(71) Applicant: Karlsruher Institut für Technologie, 76131 Karlsruhe (DE); Lancaster University Business Enterprises Limited, Lancaster, Lancashire LA1 4YW (GB)
(72) Inventor: RÖDDIGER, Tobias, 76131 Karlsruhe (DE); CLARKE, Christopher James, Trowbridge, BA14 9QU (GB); BEIGL, Michael, 76149 Karlsruhe (DE)
(74) Representative: Müller-Boré & Partner Patentanwälte PartG mbB

(57) **Abstract**

The present invention relates to systems for sensing a subject's ear canal barometric pressure, said systems comprising at least one sensor device comprising an in-ear plug for arrangement in the subject's ear canal, the sensor device being configured to provide sensor information comprising a barometric pressure level enclosed by the in-ear plug and the subject's ear canal; an evaluation device coupled to the at least one sensor device for evaluating the sensor information provided by the sensor device, the evaluation device being configured to determine a time course of the barometric pressure and, based on the time course of the barometric pressure, to determine a contraction of the tensor tympani muscle of the subject; and an interaction device coupled to the evaluation device, the interaction device being configured to provide means of explicit input to at least one computing device based on the determined contraction of the tensor tympani muscle of the subject. The present invention further relates to respective methods of providing explicit input to at least one computing device, as well as respective uses.

## Description

The present invention relates to systems for sensing a subject's ear canal barometric pressure, said systems comprising at least one sensor device comprising an in-ear plug for arrangement in the subject's ear canal, the sensor device being configured to provide sensor information comprising a barometric pressure level enclosed by the in-ear plug and the subject's ear canal; an evaluation device coupled to the at least one sensor device for evaluating the sensor information provided by the sensor device, the evaluation device being configured to determine a time course of the barometric pressure and, based on the time course of the barometric pressure, to determine a contraction of the tensor tympani muscle of the subject; and an interaction device coupled to the evaluation device, the interaction device being configured to provide means of explicit input to at least one computing device based on the determined contraction of the tensor tympani muscle of the subject. The present invention further relates to respective methods of providing explicit input to at least one computing device, as well as respective uses.

Subtle and discreet interaction techniques that require low effort and can be hidden from others are of increased interest in the field of human-computer interactions (HCI). Motivations for doing less in interaction include (a) to make interaction smaller and more comfortable so that they do not cause physical discomfort, (b) being always available and/or (c) to execute a secondary task, for example controlling mobile applications, without interrupting the manual primary task, for instance, driving a car. Moreover, subtle and discreet interaction techniques might become available for people having no other means of providing input to a device, e.g., quadriplegic patients. In this context, the term "intimate interfaces", meaning subtle, discreet and unobtrusive control of mobile devices, has been promoted. Systems that enable subtle interaction, but involve technology that itself is not subtle, include "Gunslinger" (two 3D cameras for barehand gestures), the "Magic Ring" (finger-worn wearable with accelerometer to detect small finger gestures, and "Bitey" (teeth clicking through a head-mounted bone microphone). Other discreet interaction techniques of note are "Itchy Nose" which employed electrooculography (EOG) sensors embedded in the frame of smart glasses to detect small finger gestures performed on the nose, and "Chewlt" - an intraoral input device that resembles an edible object and allows performing various hands-free input-operations.

Another common motivation for subtlety is to enable socially acceptable interaction, meaning to not disrupt others in the vicinity, or others in the group. Users may desire privacy, e.g. to protect private texts, passwords, or PIN entry. Taking this to extremes can mean completely hiding the fact that interaction happens at all. There are also application-specific motivations for subtle interaction: many researchers have approached discreet interaction in the context of different modalities, ranging from micro-gestures with the hand, fingers, gaze, and oral interfaces.

Earphones are widely adopted by consumers because they provide private audio channels to listen to music, podcasts, or audiobooks, to watch the latest movies and TV series whilst commuting, or to make hands-free phone calls. Earables are smart earphones with on-board sensors and real-time data capabilities which present new opportunities for interaction with mobile devices - where interactions may occur when the user is pre-occupied, or in public spaces surrounded by other people. Input techniques using "subtle" or "motionless" input gestures are desirable in mobile contexts because they take into consideration the social context of mobile device usage.

However, the use of the ear in the context of subtle and discreet interaction techniques is underexplored. Related work has explored a plethora of earable interaction and sensing techniques for human-centered applications. Existing, hand-based interaction techniques detect tap gestures on, mid-air interactions in front of, and touch gestures around the ear. Audio-based principles pointing in the ear were applied to detect changes in facial expression, to classify eating episodes, and for authentication. Electric-field and proximity sensing were also used to detect movement of the face. Interactions making direct use of the ear rely on deforming and pulling the ear, use ear wiggle as a subtle input technique, or use the ear as touch input to assist the visually impaired.

Input techniques with little to no movement avoid the inconvenience of techniques requiring large physical effort (e.g. hand gestures) and are more socially acceptable to spectators. The latter benefit also has the advantage from a user's perspective of maintaining a level of privacy over the interaction to avoid unwanted attention. Microgestures and hands-free input approaches enable users to interact with their device without disrupting other tasks they may be performing, for example manual tasks such as writing a letter or driving a car. Earables can be used to detect input by tapping the earphone itself, or as a sensing platform to detect more advanced gestures performed directly on, around, or in front of the ear. The earable platform also presents unique opportunities for interaction. However, the use of such interactions raises social acceptability issues and there is large scope for the exploration of more discreet methods of input using the earable medium.

Accordingly, the technical problem underlying the present invention is the provision of means for providing subtle, discreet and hands- and eyes-free input to a computing device.

The solution to the above technical problem is achieved by the embodiments characterized in the claims.

In particular, in a first aspect, the present invention relates to a system for sensing a subject's ear canal barometric pressure, comprising:
at least one sensor device comprising an in-ear plug for arrangement in the subject's ear canal, the sensor device being configured to provide sensor information comprising a barometric pressure level enclosed by the in-ear plug and the subject's ear canal;
an evaluation device coupled to the at least one sensor device for evaluating the sensor information provided by the sensor device, the evaluation device being configured to determine a time course of the barometric pressure and, based on the time course of the barometric pressure, to determine a contraction of the tensor tympani muscle of the subject; and
an interaction device coupled to the evaluation device, the interaction device being configured to provide means of explicit input to at least one computing device based on the determined contraction of the tensor tympani muscle of the subject.

Sensor devices that can be used in the context of the present invention are not particularly limited and are known in the art. Exemplary sensor devices that can be used in this respect are the Bosch BME280, BMP280, BMP380 and BME680 pressure sensors, the Maxim MPL3115A2 pressure sensor, and the Infineon DPS310 pressure sensor.

The in-ear plug used in connection with the sensor device in the system of the present invention can be selected from the group consisting of a soft silicone tip, a molded earpiece based on the subject's ear canal, and an expanding foam tip.

The sensor device comprising an in-ear plug used in the system of the present invention is for arrangement in the subject's ear canal, *i.e.,* it can be introduced into the subject's ear canal in a manner that the subject's ear canal and the in-ear plug form a closed three-dimensional space defined by the ear canal and the in-ear plug that is introduced into the ear canal. However, it is not required, and usually not the case, that said closed three-dimensional space is sealed in a perfectly air-tight manner.

Preferably, the system of the present invention comprises two sensor devices, preferably but not necessarily two identical sensor devices, for arrangement in each of the subject's ear canals, respectively. Accordingly, the evaluation device of the system of the present invention can be configured to determine the time course of the barometric pressure in one or, in case two sensor devices are present, in both of the subject's ear canals.

The tensor tympani muscle sits in the human middle ear and actuates the tympanic membrane during the middle ear reflex. The tensor's subconscious contraction accompanies vocalization and swallowing, or expecting a startling sound, and the ability to voluntarily contract the tensor tympani muscle has been discussed for over one hundred years. Due to the vibrations induced by the tensing muscle, a rumbling sound can be heard during contraction, and those who can voluntarily contract the muscle often describe it as flexing, activating, or moving a muscle inside their ear. The sound might be imitated by firmly tensing one's fist and pressing it on the ear which creates a comparable dull rumbling sound. Little information has been available about the prevalence of the ability to consciously control the tensor tympani muscle. In the present invention, an online questionnaire was conducted to provide insights into the prevalence of voluntary contraction, and explore the applicability and constraints of an input technique that relies on voluntary tensor tympani control.

In the past, the medical field documented multiple principles to sense tensor tympani muscle contraction. Electromyography is the most invasive method as it requires surgery for the placement of electrodes on the tensor. Otologists commonly measure the acoustic impedance of the ear which might also be used to detect tensor contraction. However, the technique requires playing an 800 Hz probing tone which might create discomfort. It is also possible to use a camera otoscope to detect contraction. However, the present invention opts against camera-based technology because of power consumption, computational complexity, occlusion (e.g. ear wax), and the need to focus the camera on the eardrum. Alternatively, in accordance with the present invention, in-ear barometry can be used to measure the displacement of the eardrum during contraction through pressure changes in the sealed ear canal. This approach is used in the present invention because it does not require playing a constant tone, is cheap to realize with off-the-shelf components, and can be incorporated into a pair of earphones.

Specifically, the evaluation device of the system of the present invention can be configured to determine, based on the time course of the barometric pressure in one or both of the subject's ear canals,
(i) a singular tensor tympani contraction event, wherein a negative pressure peak below a threshold of the baseline pressure reading after contraction is followed by a positive peak above a threshold of the baseline pressure reading after relaxation within a defined time threshold;
(ii) a repeated tensor tympani contraction event, wherein a singular tensor tympani contraction event as defined in (i) occurs two or more times in a row; and/or
(iii) a prolonged tensor tympani contraction event over a defined time threshold, wherein a negative pressure peak in the baseline pressure below a threshold after contraction is followed by a neutral pressure similar to the baseline pressure reading within a threshold while holding the muscle contracted and then a positive peak above a threshold of the baseline pressure after relaxation.

Accordingly, a singular tensor tympani contraction event can be detected as a negative pressure peak below a threshold of the baseline pressure reading after contraction that is followed by a positive peak above a threshold of the baseline pressure reading after relaxation within a defined time threshold; a repeated tensor tympani contraction event can be detected when a singular tensor tympani contraction event occurs two or more times in a row; and a prolonged tensor tympani contraction event over a defined time threshold can be detected as a negative pressure peak in the baseline pressure below a threshold after contraction that is followed by a neutral pressure similar to the baseline pressure reading within a threshold while holding the muscle contracted and then a positive peak above a threshold of the baseline pressure after relaxation.

Preferably, the evaluation device of the system of the present invention can be configured to determine, based on the time course of the barometric pressure in one or both of the subject's ear canals, a singular tensor tympani contraction event as defined above and/or a repeated tensor tympani contraction event as defined above. In this context, repeated tensor tympani contraction events can comprise singular tensor tympani contraction events that are repeated any number of times, e.g. two, three, four, five times or more, wherein two- or threefold repetitions are particularly preferred.

In this context, tensor tympani muscle contractions, e.g. repeated tensor tympani contraction events as defined above, can be coinciding, or synchronized, with external stimuli such as a metronome or visual cue. This can for example be used to select from a set of options presented as different stimuli that have different frequencies or rhythms.

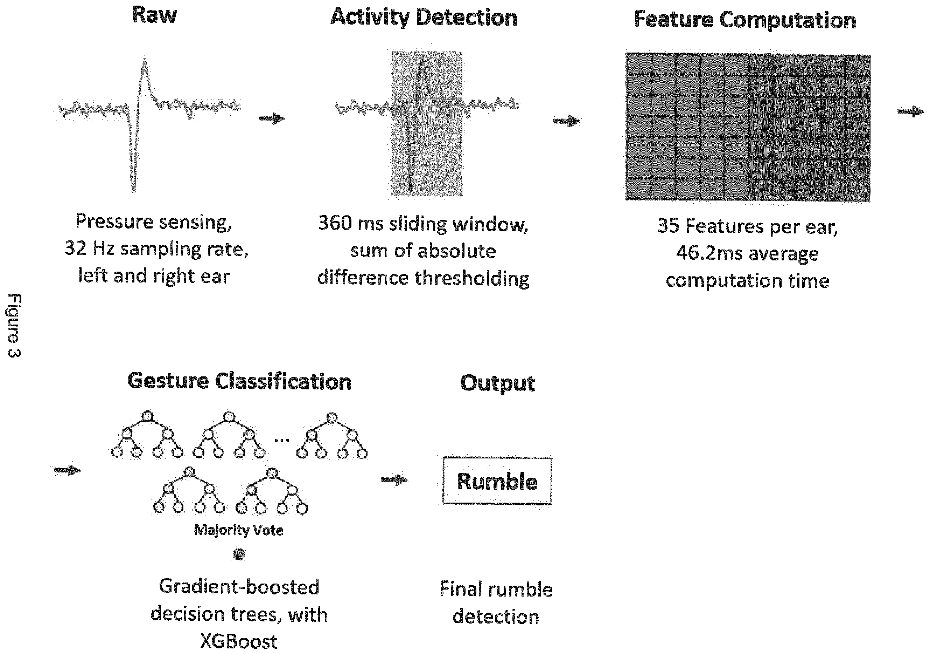

According to the present invention, contractions of the tensor tympani muscle, e.g. singular tensor tympani contraction events as defined above, repeated tensor tympani contraction events as defined above, or prolonged tensor tympani contraction events as defined above, are recognized by the evaluation device of the system of the present invention by way of applying a threshold detection algorithm and a feature-based machine learning classifier (Figure 3).

Preferably, detection of tensor tympani contraction activity uses a 360 ms sliding window with a step size of 120 ms to decide if a window of the pressure signal contains a respective activity. However, other sliding window sizes, as well as respective other step sizes of > 0 ms and ≤ the respective window size can be chosen, as known in the art. The evaluation device then computes the sum of absolute difference (SAD) within each window, and those with an SAD above 20 Pascal (PA) are flagged as containing activity. However, other SAD values can be chosen in linear proportion to the chosen sliding window size. An activity is considered complete and passed to the classifier after e.g. four consecutive windows without activity.

Once an activity is detected, it is passed to the classification stage to determine whether a tensor tympani contraction event has been detected. Before being passed to a classifier, features are extracted from the signal. Preferably, a set of 70 features (35 features per ear canal) is extracted and passed to the classifier.

Accordingly, the evaluation device of the system of the present invention can be configured to determine, based on the time course of the barometric pressure in one or both of the subject's ear canals, one or more or all features of said time course, selected from the group consisting of a positive pressure peak above a threshold of the baseline pressure, a negative pressure peak below a threshold of baseline pressure, the rising or falling time of a respective positive or negative pressure peak, the slope of a respective positive or negative pressure peak, the correlation of the barometric pressure at a given point in time as determined in both of the subject's ear canals, the number of respective positive and/or negative pressure peaks, the mean distance between respective positive and/or negative pressure peaks, the absolute difference of the first two of respective positive and/or negative pressure peaks, the minimum and/or maximum values of respective positive and/or negative pressure peaks, the location of respective positive and/or negative pressure peaks over time, the absolute difference between respective positive and/or negative pressure peaks, the ratio of maximum and/or minimum of respective positive and/or negative pressure peaks to the mean pressure level, the minimum slope and/or maximum slope and/or intercept of respective positive and/or negative pressure peaks over a five sample rolling window, the sum of absolute differences (SAD) of the pressure signal, the auto-correlation of the pressure signal, the variance of quantile changes of the pressure signal, the spectral welch density of the pressure signal, the continuous wavelet transform of the Ricker wavelet of the pressure signal, and the FFT coefficients of the pressure signal.

According to the present invention, a tensor tympani contraction event can be calculated by automated means from the above one or more or all features by a machine learning classifier.

Classifiers used in the context of the present invention are preferably selected from the group consisting of a decision tree, a support vector machine, a random forest classifier, and a neural network, and are more preferably selected from the group consisting of a radial basis function (RBF) kernel support vector machine (SVM), k-nearest neighbors (kNN), random forest (RF), and gradient boosting (XGBoost), as known in the art, wherein the classifier XGBoost is particularly preferred.

The interaction device of the system of the present invention can be configured to use the determined activities of the subject, e.g. a determined tensor tympani contraction event as defined above, to provide means of explicit input to one or more computing device(s) to effect control of said computing device(s).

Respective computing devices are not particularly limited and include any device to which the provision of input by a user is desired. Preferably, the computing device is an earphone device or earable device as defined above.

Accordingly, the system of the present invention can be integrated in a respective computing device, e.g. in an earphone device or in an earable device.

In a second aspect, the present invention relates to a method of providing explicit input to at least one computing device, comprising the steps of
(i) detecting a contraction of the tensor tympani muscle of a subject, and
(ii) using a detected contraction event as the explicit input to the at least one computing device.

In a related third aspect, the present invention relates to the use of the contraction of the tensor tympani muscle of a subject as explicit input to at least one computing device.

In these second and third aspects of the present invention, all relevant definition and limitations defined for the system according to the first aspect of the present invention apply in an analogous manner.

In particular, the contraction of the tensor tympani muscle of the subject is preferably detected using the system according to the first aspect of the present invention.

Accordingly, a contraction event of the tensor tympani muscle of a subject that is detected or used according to the above second or third aspect of the present invention can be
(i) a singular tensor tympani contraction event, wherein a negative pressure peak below a threshold of the baseline pressure reading after contraction is followed by a positive peak above a threshold of the baseline pressure reading after relaxation within a defined time threshold;
(ii) a repeated tensor tympani contraction event, wherein a singular tensor tympani contraction event as defined in (i) occurs two or more times in a row; and/or
(iii) a prolonged tensor tympani contraction event over a defined time threshold, wherein a negative pressure peak in the baseline pressure below a threshold after contraction is followed by a neutral pressure similar to the baseline pressure reading within a threshold while holding the muscle contracted and then a positive peak above a threshold of the baseline pressure after relaxation.

Accordingly, a singular tensor tympani contraction event can be detected as a negative pressure peak below a threshold of the baseline pressure reading after contraction that is followed by a positive peak above a threshold of the baseline pressure reading after relaxation within a defined time threshold; a repeated tensor tympani contraction event can be detected when a singular tensor tympani contraction event occurs two or more times in a row; and a prolonged tensor tympani contraction event over a defined time threshold can be detected as a negative pressure peak in the baseline pressure below a threshold after contraction that is followed by a neutral pressure similar to the baseline pressure reading within a threshold while holding the muscle contracted and then a positive peak above a threshold of the baseline pressure after relaxation.

Preferably, a contraction event of the tensor tympani muscle of a subject that is detected or used according to the above second or third aspect of the present invention is a singular tensor tympani contraction event as defined above and/or a repeated tensor tympani contraction event as defined above. In this context, repeated tensor tympani contraction events can comprise singular tensor tympani contraction events that are repeated any number of times, e.g. two, three, four, five times or more, wherein two- or threefold repetitions are particularly preferred. As used herein, the term "comprising"/"comprises" expressly includes the terms "consisting essentially of "/"consists essentially of" and "consisting of "/"consists of", *i.e.,* all of said terms are interchangeable with each other herein.

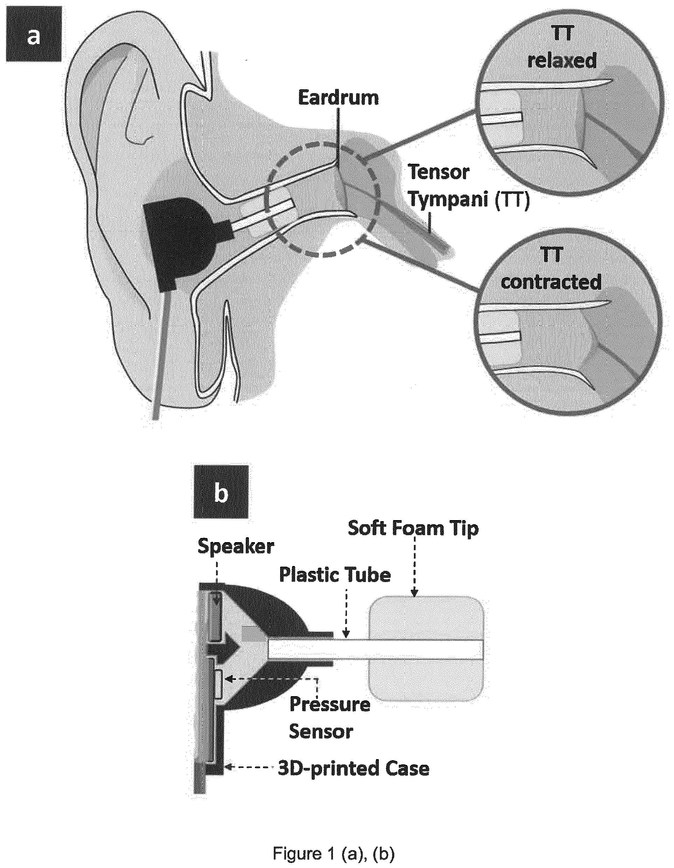

The present invention introduces "EarRumble", an interaction technique which uses contraction of the tensor tympani muscle inside the middle ear (Figure 1 (a)). The tensor tympani muscle is the second smallest muscle found in the human body, and is used for dampening loud noises. Interestingly, a subset of the population has voluntary control over the contraction of the tensor tympani muscle which causes in-ear vibrations when contracted. As the tensor tympani contracts, it tightens the eardrum and the volume encapsulated in the ear canal rises, resulting in a reduction in pressure. EarRumble measures pressure changes within the sealed ear canal to unobtrusively detect contraction of the tensor tympani (Figure 1 (b)).

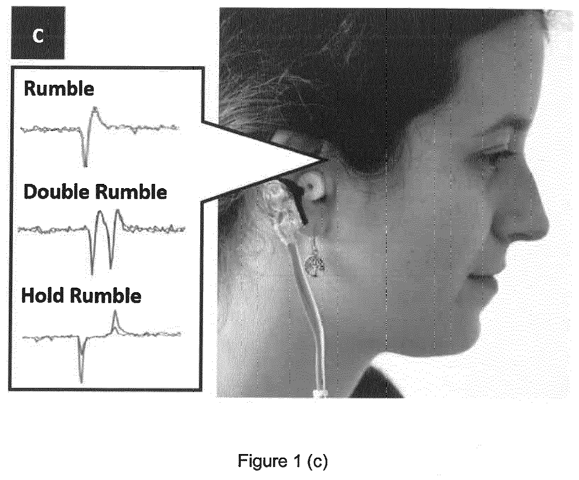

The present invention further explores how this phenomenon, also known as ear rumbling, can be exploited for interaction with mobile devices using simple, discreet gestures - however, not everyone has voluntary control of the tensor tympani muscle. To inform how many people could hope to use ear rumbling for interaction, and to demonstrate that rumbling is a viable opportunity for others to pursue, the prevalence of rumbling was investigated through an online questionnaire (N=198) which revealed that 43.2% of respondents had the ability to voluntarily contract the tensor tympani. As contraction of the muscle is unlikely to occur voluntarily on a regular basis, it is unclear what level of control users have over the muscle for the purposes of interaction. To address this, initial insights into the complexity of the interaction design space was provided through analysis of different ear rumble gestures (e.g. sequential rumbles, holding the rumble) and gather feedback on user perception of how easy and comfortable they are to perform on demand to determine their viability. Data from participants with the ability to ear rumble (N=16) was collected, and the characteristics of three different ear rumble gestures was analyzed (Figure 1 (c)). Using the data collected, a recognition pipeline which detects ear rumble gestures from everyday activities that may also induce pressure changes in the ear canal was developed, with up to 95% accuracy with real-time performance. Finally, it was explored how EarRumble can be used for interaction in two exemplar applications (receiving phone calls and an audio player) using three manual, dual task application scenarios that one might face when using earables in everyday life. Users (N=8) provided positive feedback, describing how the EarRumble technique felt "magical" and "telepathic", and highlighting how EarRumble is a low-effort, hands- and eyes-free input technique.

In sum, the present invention provides the following advantageous contributions: (i) EarRumble, a hands- and eyes-free, discreet input technique based on voluntary control of the tensor tympani muscle found in the middle-ear, and sensed through in-ear barometry; (ii) an indication of how prevalent ear rumbling is, how easy it is to perform on demand, and how comfortable it is through an online questionnaire; (iii) data-driven insights into how well users can contract the tensor tympani muscle in the context of interaction; and (iv) insights into how EarRumble can be used for interaction, grounded in real-world applications involving dual task scenarios.

The figures show:
Figure 1:
   The present invention provides a technique that uses "ear rumbling" for interaction. (a) The tensor tympani muscle can be contracted voluntarily which displaces the eardrum and induces a pressure change within the sealed ear canal; (b) Custom-built earables detect ear rumbling using an in-ear pressure sensor; (c) Eyes- and hands-free discreet input can be provided by performing different rumbling gestures by voluntarily contracting the tensor tympani muscle.
Figure 2:
   The custom-built device used to realize the EarRumble technique of the present invention. Each earbud contains a speaker, which provides the usual audio capabilities of a pair of headphones, and a BME280 pressure sensor which is used to detect the ear rumble gestures by measuring the changes of pressure inside the ear canal. The soft foam tip and hot glue provides an air-tight seal within the ear canal. A custom 3D-printed enclosure houses the components and provides two separate channels for the speaker and pressure sensor.
Figure 3:
   The EarRumble recognition pipeline - the raw pressure signals are sampled at 32 Hz from both ears. A sliding window of 360 ms is used to detect if activity is present in the signal. 35 features are sampled from each ear, which are then passed to a gesture classifier to identify if the signal contains an ear rumble.
Figure 4:
   The median and inter-quartile range (IQR) for online dataset from Example 2 and lab participants from Example 3 for the two Likert items - ease to perform on demand and comfort for performing - for (a) a rumble, (b) rumbling in quick succession, and (c) changing the duration of rumbles (1: strongly disagree SD, 7: strongly agree SA). Figure (d) shows the fractions of required secondary movement during different rumbling variations.
Figure 5:
   Zero-shifted pressure readings measured in both ears for the different activities. The four rumbling variations show how the pressure readings spike downwards with initial muscle contraction and indicate another peak in the opposite direction after relaxation - creating distinct patterns for rumbling variations. The four noise activities have different characteristics.
Figure 6:
   Likert item responses from the 16 lab participants after performing rumble gestures in silence and with music.
Figure 7:
   (a)-(c) show the distribution of start and completion time for the different rumbling variations. Interestingly, a double rumble does not take twice as long as a single rumble whereas (d) and (e) show how the first rumble is faster to execute than the second, (f) shows how for repetitive rumbling the mean cycle time was significantly longer than during double rumbling.
Figure 8:
   The confusion matrix shows how short, subsampled noise activities can confuse the classifier for single rumble detection and *vice versa.*

The present invention will be further illustrated by the following examples without being limited thereto.

### Examples

### Example 1

### "EarRumble" - the system of the present invention

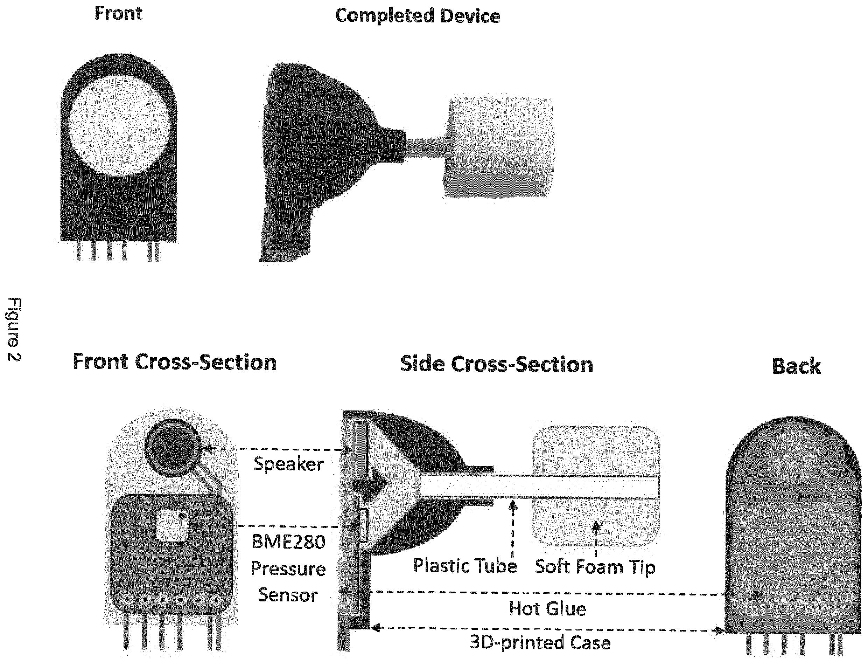

The present invention introduces EarRumble, an interaction technique based on the voluntary contraction of the tensor tympani muscle found in the human middle ear - a phenomenon also known as ear rumbling. The contraction of the tensor tympani is detected using in-ear barometry, *i.e.,* measurement of pressure changes within the ear canal, using a custom-built earable consisting of commercial off-the-shelf (COTS) components and a custom 3D-printed enclosure (Figure 2). A thresholding detection algorithm and feature-based machine learning classifier are applied to recognize three basic ear rumbling gestures from the raw pressure signals (Figure 3).

### Concept:

Figure 1 (a) illustrates the underlying principle of the EarRumble interaction technique. Upon contraction of the tensor tympani muscle, the eardrum displaces inward. As the soft foam earcaps worn by the user seal the ear canal, the volume increases while the encapsulated air remains constant which results in a pressure drop. After the relaxation of the muscle, the eardrum returns to its original position. This leads to a pressure wave that is pushed outwards of the ear canal to produce a positive pressure peak, as shown in the Rumble signal in Figure 1 (c). As the sealing of the ear canal is not perfectly air-tight, the pressure equalizes overtime. Therefore, holding the tensor tympani contracted does not yield a constant low pressure reading, however releasing the muscle still produces a sufficiently pronounced pressure peak in the opposite direction, as shown in the Hold Rumble signal in Figure 1 (c). The changes of pressure are utilized to derive contraction events of the muscle which can be measured with a standard, off-the-shelf pressure sensor. It is assumed that the tensor tympani can be in one of two states - relaxed or contracted - and the present exploration was focused on the use of three basic ear rumble gestures:
- Single rumble - a quick contraction of the tensor tympani
- Double rumble - two contractions of the tensor tympani in quick succession
- Hold rumble - contraction of the tensor tympani for approximately one second

### Hardware:

Figure 2 illustrates the assembly of a custom-built device according to the present invention. To realize in-ear barometry, the Bosch BME280 pressure sensor (2.5 × 2.5 × 0.93 mm) sampling at 32 Hz is used. The speaker was removed from a pair of commercially available earbuds (Sony MDR-EX110LP) and a custom earplug case was 3D-printed that encapsulates the different components in a single device. The case splits the enclosed air into two channels, one directed towards the speaker and the other towards the pressure sensor. This was done to minimize the volume of air enclosed within the ear canal, and maximize the change in pressure. To ensure tight sealing of the ear canal, foam earplugs (Etymotic Research Disposable eartip ER1-14A, 13mm diameter) was used. Before insertion, users firmly squeeze the tip, which then expands within the ear to create a tight seal. To increase air-tightness further and to keep the electronic components in place, the 3D-printed case was sealed by applying hot glue on the backside of the components. The manufacturing process is the same for left and right earbuds, except that the device uses stereo sound which plays the respective channel on either of the ears. The pressure sensors in the earphones connect to an ESP32 MCU breakout board using I2C, and data is transferred to a PC using serial communication. The audio signal connects to the same workstation using the aux connector.

### Recognition pipeline:

Figure 3 illustrates the final recognition pipeline of EarRumble. Other classifiers and their performance are evaluated in Example 3.

### Detection:

Activity detection uses a 360 ms sliding window with step size of 120 ms to decide if a window of the pressure signal contains activity. The detector computes the sum of absolute difference (SAD) within each window, and those with an SAD above 20 Pascal (PA) are flagged as containing activity. The threshold value was deduced from different rumble activity and non-activity samples that were collected in Example 3, and ensures that > 95% of all samples are correctly detected. A correct detection was defined as one in which more than 75% of the gesture was detected as activity. An activity is considered complete and passed to the classifier after four consecutive windows without activity.

### Classification:

Once an activity is detected, it is passed to the classification stage to determine whether an ear rumble has been detected. Features are extracted from the signal, before being passed to a gesture classifier. Four different classifiers were evaluated: a radial basis function (RBF) kernel support vector machine (SVM), k-nearest neighbors (kNN), random forest (RF), and gradient boosting (XGBoost). The XGBoost classifier yields the highest overall accuracy at 0.95. More details are presented in Example 3.

For classification, self-defined features were evaluated, in addition to systematically derived features from tsfresh using the data gathered in Example 3. All features are computed on the zero mean-shifted signal to account for drift in the pressure readings. To reduce the initial collection of 1618 features systematically, importance selection with XGBoost feature importance scores was applied. If a feature was deemed relevant for the left or right ear only, it is computed for both ears to account for different laterality conditions. As a result, a set of 70 features (35 per ear) is used for feature extraction, which are then passed to the classifier. The average computation time of all features across all collected samples was 46.2 ms (Intel Core i7-9700KF 8 × 3.7 GHz).

For replicability, the features used include the number of, and the mean distance between peaks (both negative and positive), the absolute difference of the first two peaks, and the minimum and maximum values of the signal and also their locations. Additionally, the absolute difference and ratio of maximum to mean, and also the minimum and maximum slope and intercept over a five sample rolling window. The sum of absolute differences (SAD) of the whole signal was used and also SAD of four even sequences that each sample was cut into. Finally, auto-correlation of the signal (lag: 2, 5, 9, 32), the variance of quantile changes (ql - qh: 0.2 - 1.0, 0.0 - 0.8), the spectral welch density (c: 2, 5), the continuous wavelet transform of the Ricker wavelet (c / w: 0 / 20, 2 / 2, 4 / 5, 8 / 20) and the FFT coefficients of the signal (c: 1, 2) were used.

### Example 2:

### Prevalence of tensor tympani muscle control

Although not part of the acoustic reflex, the involuntary contraction of the tensor tympani muscle (tympani reflex) helps to prevent ear damage from loud noises. However, not everyone can voluntarily contract the muscle to cause in-ear vibrations, and there is currently no data about the prevalence of ear rumbling, nor how well people can voluntarily control the tensor tympani muscle. In this section, a large sample of participants was surveyed remotely using an online questionnaire, predicated on the basis that voluntary contraction of the tensor tympani can be self-reported due to audio feedback during the contraction of the muscle.

Insight into what proportion of the population can voluntarily control the tensor tympani muscle was sought - to inform how many people could hope to use ear rumbling for interaction. Of those who can voluntarily control contraction of the tensor tympani, deeper insight was sought with respect to the level of control, isolation, and laterality when performing the ear rumbling. The level of control was surveyed that participants have when contracting the muscle to inform the potential complexity of the interaction design space. The level of discreetness afforded by ear rumbling as an interaction technique was investigated, which is dependent upon whether the tensor tympani can be contracted in isolation of other physical movements or facial gestures. Finally, it was attempted to discover whether participants can perform ear rumbling in one ear or both, which informs whether signals from the ears can be treated independently or in combination.

### Design and procedure:

To minimize non-response bias - where those who cannot ear rumble find the questionnaire less appealing - participants were recruited using neutral, context-free online ads and a social media post (Twitter) that did not reveal information on the nature of the study. Participants could use any device with browsing capabilities to fill in the survey, and no reward was offered for participating in the study. Firstly, participants were presented with the information sheet and relevant consent forms. Participants were not admitted to the study if they self-reported acute ear-related health conditions or were not at least 18 years of age.

The first page introduced the concept of "ear rumbling" by describing the contraction of the tensor tympani muscle. This was illustrated using an animated image of the human ear and a short textual description based on observations from people who have voluntary control, related work reporting the phenomenon, and by talking to an ear, nose, and throat doctor. Participants were then instructed to move to a quiet environment and to remove headphones, with explicit instructions for those who wear hearing aids to leave them on. Participants were then asked to attempt to activate, flex, or move the muscles inside their ear to produce a rumbling sound. In addition, clarification was added that this is not to be confused with ear wiggling, and that some people can only perform ear rumbling when performing other actions (e.g. when yawning, swallowing, with closed eyes, or with the mouth open). Participants were then asked whether they could hear a "rumble or vibration", or a "clicking, crackling or popping" sound, or both. It was asked about the clicking, crackling, or popping sound as such sounds might be induced by opening the Eustachian tube for pressure exchange, rather than contraction of the tensor tympani muscle.

Participants who reported that they can perform ear rumbling, either with or without crackling noises in addition, were asked which ear they could hear the rumbling in and whether they could perform the rumbling independently of other actions, such as closing the eyes, blinking, or swallowing. They were also asked to complete two 7-point Likert items (1: strongly disagree, 7: strongly agree), one asking whether the rumbling is easy to perform on demand, the other asking whether it is comfortable to perform. To investigate the level of control users have of contracting the muscle, participants were asked whether they could perform the rumbling in quick succession (e.g. one ear rumble directly after another), and whether or not they could control the duration of the rumbling (e.g. hold an ear rumble for one second). The order in which these questions were presented was counterbalanced in the event that performing one movement made it more difficult to perform the other. If participants answered yes to either of these, they were asked the same two Likert items regarding ease of performance on demand and comfort, in addition to whether any additional action was required for the rumbling. The whole survey took around seven minutes to complete.

To validate the results from the online questionnaire, the same questionnaire was given to sixteen participants who completed the questionnaire as part of an in-person evaluation in Example 3. These responses were validated visually using a USB otoscope and form a separate dataset. The responses were compared to the Likert items from the online participants with those from Example 3 to see if any statistically significant differences exist between the two datasets.

### Results:

208 participants completed the study, from a total of 1399 clicks on the adverts. After data cleaning, there were 192 completed data sets (110 male, 78 female, 1 other, 4 preferred not to answer, age: M = 40.1, SD = 13.7, min = 18, max = 76). Eight participants had reduced hearing abilities (4 medical and 4 self-diagnosed), three wore hearing aids on both ears, and one participant was deaf.

### Prevalence:

Out of 192 participants, 83 (43.2%) reported that they could produce a rumbling or vibrating sound on at least one ear. Using the normal approximation interval, this results in a 95% confidence interval between 36.2% to 50.2%. Of the 83 who reported that they could produce a rumbling, 18 reported that they heard a crackling, clicking or popping sound in addition to the rumbling. Out of the 18 participants who reported rumbling and popping sounds, 9 said that they can do the rumbling sound independent of the crackling. In addition to those who reported some form of rumbling, 44 participants reported a crackling sound, but no rumbling. Those who did not report a rumbling sound were not asked to complete the remainder of the study. The deaf participant did not report rumbling, however participants with reduced or no hearing ability might still be able to contract the tensor tympani, but are lacking the audible feedback loop.

### Laterality:

Of those who could rumble, 68 participants (81.9%) reported the rumbling sound on both ears simultaneously, 11 (13.3%) in isolation on the left, and 19 (22.9%) in isolation on the right ear. Overall, 14 (7.1%) participants reported that they could perform rumbling on both ears and also in isolation on one ear - suggesting a high level of control of the muscle contraction. No participants reported the ability to perform rumbling on both ears and in isolation on both sides.

### Control:

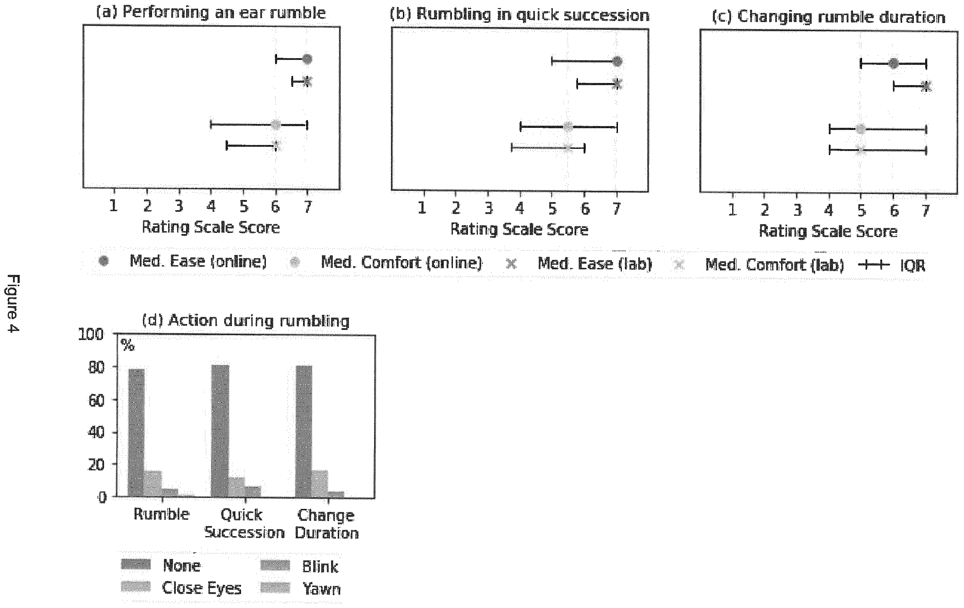

Out of all those who could rumble, 74 (89.2%) reported the ability to perform rumbles in quick succession, and 71 participants (85.5%) could change the duration of rumbles. 67 participants reported that they could both perform rumbles in quick succession and change the duration of the rumbles. Interestingly, three of the participants with reduced hearing abilities could perform all rumbling variations (1 medical, 2 self-diagnosed). Figure 4 (a) to (c) shows that participants perceived ear rumbling to be easy to perform on-demand and comfortable. A Friedman test was performed of those who reported that they could perform all types of ear rumble, which revealed a significant difference for responses about how easy ear rumbling is to perform on-demand, but no difference for the reported level of comfort across the rumble variations. Posthoc Wilcoxon rank-sign tests revealed that performing a single ear rumble was perceived to be much easier to perform on demand compared with both successive rumbles (Z=37.5, p < .01), and when holding a rumble for a prolonged duration (Z=27.5, p < .01). There was no statistically significant difference between successive rumbling or holding for a prolonged duration.

### Isolation:

The majority of participants did not have to perform secondary actions when contracting the tensor tympani muscle. Five participants required a secondary action when performing repetitive rumbling, and six participants when holding the rumbles for a longer duration. Figure 4 (d) shows the breakdown of the three types of secondary action which were required by these participants: closing eyes, blinking, and yawning.

### Comparison to lab dataset:

To validate the reliability of the Likert items administered online, the responses from the online participants were compared with responses from participants who completed the same questionnaire in-person as part of the data collection performed in Example 3. Mann-Whitney U tests showed that no significant differences existed between the responses across the three types of rumbling (single, changing duration, and successive rumbling) for either of the Likert items. Both data sets showed that participants found ear rumbling easy to perform on-demand, and comfortable (Figure 4).

### Qualitative results:

Five participants commented that they remember being able to perform ear rumbling since they were a child (P492, P556, P1320, P1809, P1709) and were surprised to learn that not everyone has that ability. Another participant said that they can recall involuntary rumbling during stressful situations (P1320). One participant expressed the urge to keep rumbling after rumbling once (P480). Another participant with mild tinnitus mentioned that the rumbling can only be perceived when the surrounding area is very quiet. One participant expressed that rumbling led to a tense feeling in the cheek muscles.

### Discussion:

These results demonstrate that a substantial proportion of those who responded had the ability to voluntarily contract the tensor tympani muscle, and generally participants reported that ear rumbling is easy to perform on demand and comfortable. It is unclear if and how the muscle contraction might be learnable for those who do not possess the ability to voluntarily contract the tensor tympani, or indeed if the muscle can be strengthened over time for those who can. Very few people required secondary actions (such as closing the eyes) to induce the ear rumble, which makes it ideal as a discreet interaction technique that is externally hard to notice. Of those who reported they could perform an ear rumble, 80.7% reported that they could change the duration of the ear rumble, and perform multiple ear rumbles in succession. Using this knowledge, it can be investigated how a simple gesture set that leverages these characteristics can provide simple input capabilities using contraction of the tensor tympani. This is explored in more depth in Example 3.

The results reported above are predicated on the ability of participants to accurately self-report the ability to voluntarily contract the tensor tympani. In Example 3, all participants (N=18) were initially recruited based on self-reporting the ability to ear rumble, which was validated using a camera otoscope and confirmed in all cases. It is also noted that the subjective responses of the in-person participants were not significantly different compared with the online survey. However, due to the nature of the online survey, it was not possible to physiologically validate the ability to voluntarily contract the tensor tympani for the remote participants, and it is important to note that the data collected does not stand as physiological evidence.

### Example 3:

### Data collection and analysis

A study was conducted to investigate the feasibility of detecting ear rumbling across a range of participants using in-ear barometry. It was attempted to understand how well users can perform ear rumbling on-demand, how comfortable they find it, and the general characteristics when performing ear rumbles to inform interaction design. Based on initial data exploration, the data collection was focused on the use of three ear rumble gestures: single, double, and hold rumble.

The participants' ability to perform repetitive sequential rumbling was also investigated, where they were asked to contract the tensor tympani multiple times in quick succession. It was sought to understand the level of control participants had over the muscle contraction, because a higher level of control opens up the opportunities for the use of switch scanning interfaces, rhythmic patterns, or beat synchronization for interaction.

It was known that tensor tympani contraction might occur during swallowing and vocalization. This begs the question as to whether pressure changes from ear rumbling compared with similar everyday activities can be accurately distinguished. With this in mind, data on the following actions which may induce pressure changes in the ear canal was also captured: opening and closing the mouth, reading out loud, drinking and swallowing, and chewing gum. The tensor tympani muscle contraction may also be induced by sound, so that ear rumbling was investigated under two conditions: in silence, and with music playing through the earphones.

### Participants and apparatus:

18 participants were recruited through e-mail and a university Facebook group. Prior to the study, participants self-reported that they could perform ear rumbling, which was validated using an otoscope and confirmed in all cases. The study was conducted according to national COVID-19 regulations and within the university's safety guidelines. Participants wore a custom-built, in-ear pressure sensing device according to the present invention on both ears (cf. Example 1, section "Hardware"). Each participant's outer ear canal was measured using a caliper, and two participants were excluded because of insufficient sealing of the ear canal with the ear buds (> 14 mm external ear canal diameter). The final dataset consists of 16 participants (13 male, 3 female, Age: M = 24.7 SD = 2.63, Ear canal width: M = 8.0 mm SD = 1.6 mm, Ear canal height: M = 11.2 mm SD = 1.8 mm). All participants reported that they had no hearing loss, and none of the participants wore a hearing aid.

### Design and procedure:

Participants began the study by completing the same questionnaire about their ability to ear rumble featured in Example 2, and all participants reported that they had not participated in the online questionnaire in Example 2. Following this, the researcher measured the ear canal and verified that the ear rumbling reported by participants was caused by contraction of the tensor tympani muscle. This was validated by visual inspection of the eardrum with a Teslong USB digital in-ear camera otoscope. Participants were then asked to wear the EarRumble earphones and ensure a tight fit so that pressure differences could be detected.

Participants performed nine activities - four ear rumbling gestures (single, double, hold, repetitive), four everyday activities (opening/closing mouth, reading out loud, drinking, and chewing gum), in addition to an activity where users were asked to do nothing. A display was used to indicate which activity the participant should perform. Each activity was preceded by a five second on-screen countdown, after which participants were instructed to execute the activity in a five second window. Participants were asked to perform the rumbling gestures immediately and as quickly as possible after the countdown. Data was recorded from the start of the countdown, until the five seconds of the activity had elapsed. After each activity there was a five second break. After performing one of the rumbling activities, participants completed two 7-point Likert items (1: strongly disagree to 7: strongly agree):
- *Ease-on-demand:* The [rumbling activity] was easy to perform on demand.
- *Comfort:* The [rumbling activity] was comfortable to perform.

The present study follows a within-subject design, where all participants performed all activities. Participants performed the activities in two blocks: one in silence, and one with music playing (Symphony No. 5 by Ludwig van Beethoven). The order of the blocks was counterbalanced, *i.e.,* half the participants performed in silence and then with music, and half *vice versa.* For each block, participants were asked to perform all rumble gestures, prior to performing all of the everyday activity tasks in a fixed order: open/close mouth, vocalization, drink and swallow, chew gum, and finally do nothing. The order in which the rumble activities were presented to participants was counter-balanced using a balanced Latin square design. For each activity, participants had a training phase prior to the data recording, in which each activity was repeated five times. The study lasted approximately 60 minutes, and participants received a bag of candy as a reward for their participation.

### Data labeling:

The collected data were labeled by hand to be used for analysis and development of the EarRumble recognition pipeline. One researcher precisely identified the start and end time of single and double rumbles, and a second researcher verified the labels. The start time was labelled for the hold rumbles, however the exact end time could not be identified from the recorded data for the majority of samples because it was observed that the pressure equalized over time and there were no visible features to identify the end. In total there were 79 hold rumbles that were labelled by hand (49.3%). For repetitive and double rumbling, the peaks of each rumble were labeled to extract the periodicity. The peak detection was automated, using the Python Scipy signal processing library, and hyperparameters were fine-tuned if they did not fit the individual samples. The accuracy of the peak detection was verified by visual inspection for all samples.

### Results:

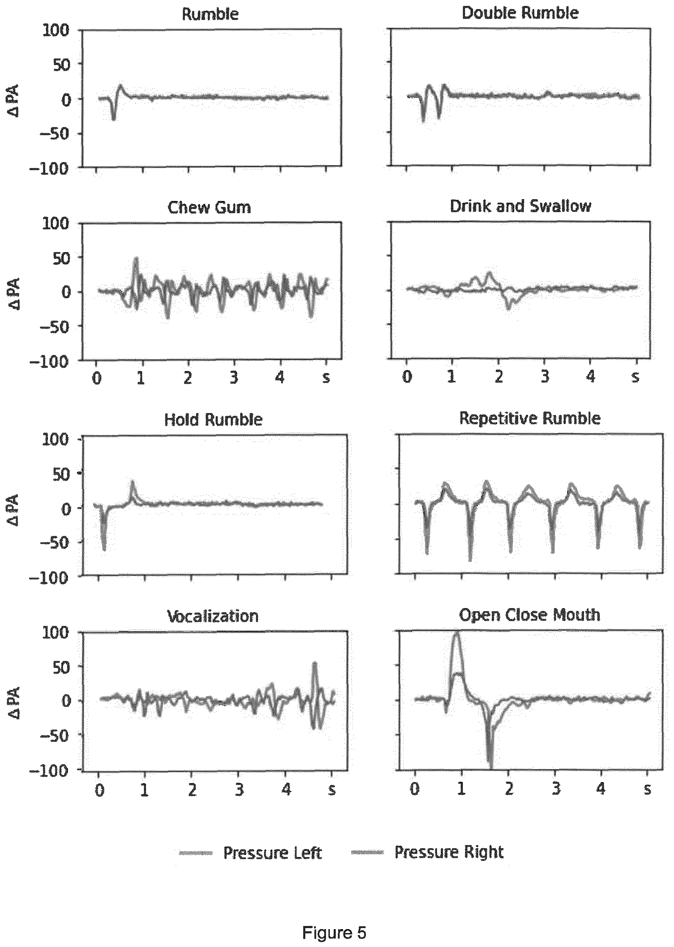

The following section explores the participants' perception of ear rumbling and the characteristics of how users contract the tensor tympani muscle when performing different gestures. Figure 5 shows samples for all the different activities that participants performed during the study. All statistical results are reported as significant if p < .05, unless using Bonferroni correction to account for multiple comparisons.

### Questionnaire responses:

In the beginning, the responses to the questionnaire that was also administered in Example 2 were investigated. All participants correctly reported that they could rumble, which was validated visually with an otoscope. Five participants self-reported that they heard crackling in addition to the ear rumbling. Fourteen people reported that they could rumble in both ears, with the remaining two reporting that they could only rumble in their right ear. Three people out of the fourteen reported that they could perform ear rumbling in isolation in one ear in addition to both ears (1 right, 2 left). No participants reported that they could perform ear rumbling in both ears and in isolation in both left and right ears. Visual inspection of the pressure sensor data confirmed the laterality that participants reported, with little to no pressure changes being observed when participants reported rumbling in only one ear. All participants reported that they could change the duration of the ear rumble, and that they could repeat the rumble in quick succession. Similarly, all participants reported that they could contract the tensor tympani muscle without having to perform other activities, such as closing their eyes or opening the mouth.

### User perception:

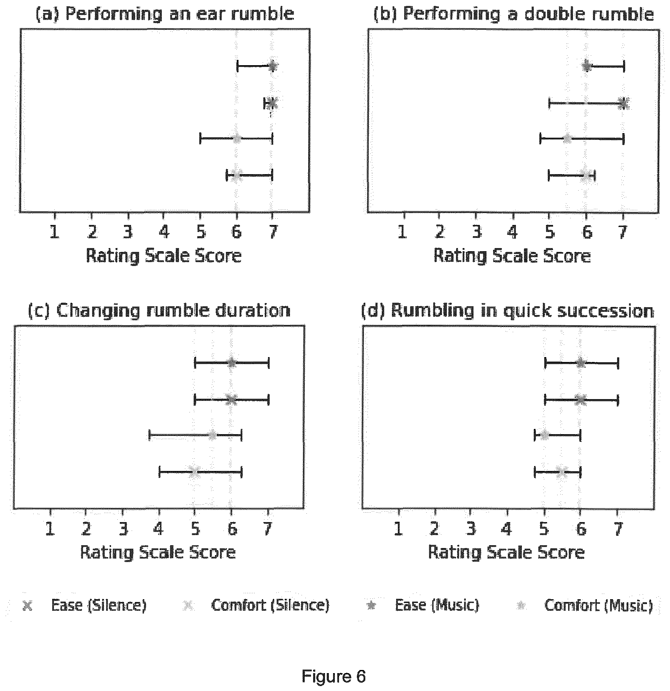

Figure 6 shows the median and inter-quartile ranges for the Likert items participants completed during the data collection (cf. Figure 4 for the Likert responses for the lab participants' responses to the questionnaire from Example 2). Wilcoxon signed-rank tests showed that there was no significant differences for responses between the music or silence conditions across all the different rumble gestures. In general, participants reported that the ear rumbling gestures were easy to perform on demand, and comfortable. A Friedman test was performed on the "easy to perform on demand" and "comfortable" Likert items to see whether participants' perception was consistent across the different types of ear rumbling gestures. Participant results were significantly different for both the easy to perform (*χ2* (3) = 11.79, *p* = .008), and comfortable Likert items (*χ2* (3) = 10.22, *p* = .017). Pairwise comparisons using the Wilcoxon signed-rank test were performed with a Bonferroni correction for multiple comparisons. These revealed that participants found it significantly easier to perform a single rumble compared with holding the rumble for approximately 1 second (Z = 4.0, p = 0.0079). No other results were significant after Bonferonni correction.

### Analysis of ear rumble gestures:

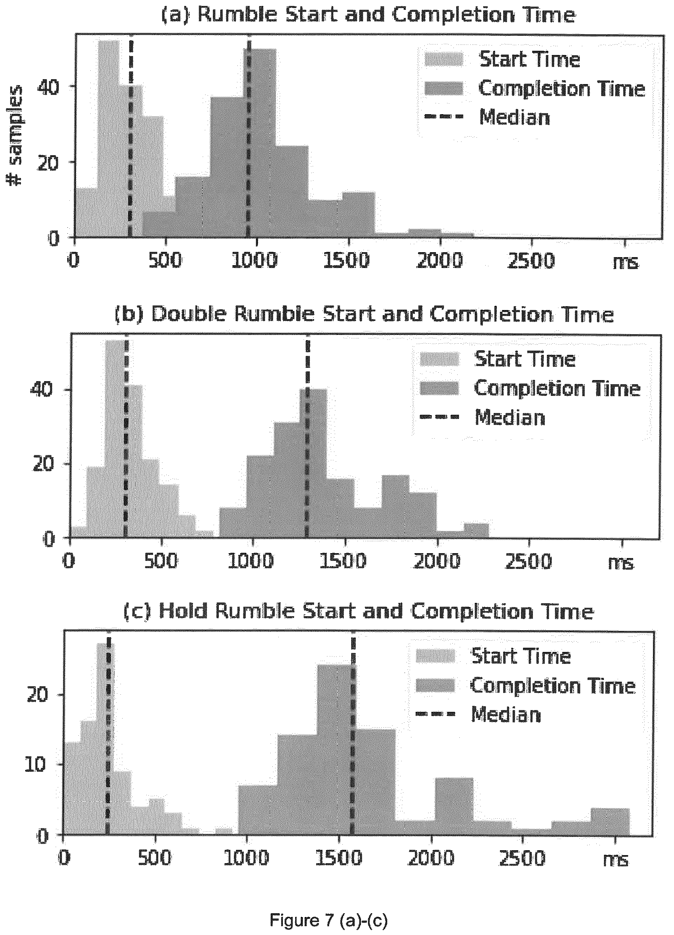

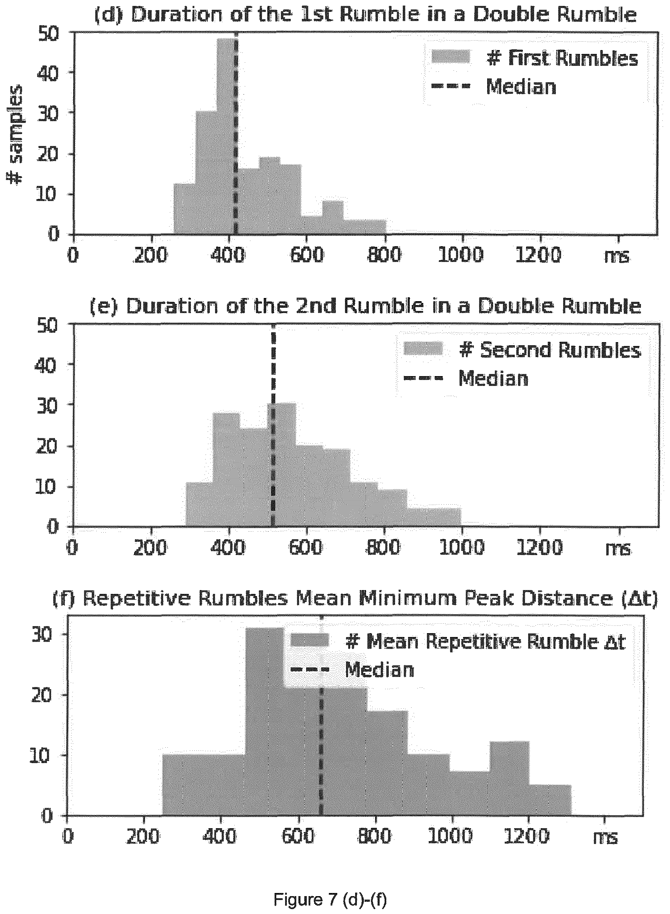

Figure 7 (a)-(c) shows the time to start the ear rumble when performing each of the three different ear rumble gestures (rumble, double rumble, and hold rumble). The start time incorporates the participants' reaction time to the visual stimulus, and the time taken to start the gesture in response. As participants were presented with a five second countdown, the reaction time would be expected to be smaller than that of a random stimulus. Statistical tests were performed to see if there was a significant difference between the reaction times across the gestures, however due to the difficulty in extracting ground truth labels for the hold rumble only single versus double rumbles were compared. Shapiro-wilks test of normality revealed both distributions were not normally distributed (p < .05), and hence the Wilcoxon signed-rank test was used. There was no significant difference between the start time for the single rumble (Median = 308 ms) compared with the double rumble (Median = 308 ms).

A Wilcoxon signed-rank test (Z = 407.5, p < .001) showed that a single rumble (Median = 958 ms) was significantly quicker to perform than a double rumble (Median = 1301 ms) - as to be expected. Interestingly, when analyzing the duration of each rumble gesture, it was observed how the double rumble (Median: 1010 ms) takes only 48% longer than a single rumble (Median: 684 ms). Figure 7 (d) and (e) shows a histogram of the durations for the first and second rumble in the double rumble gesture. By comparing the time difference between rumbles, it is seen how the first rumble when performed in the double rumble gesture is performed significantly quicker (Median = 419 ms) than the second rumble (Median = 516 ms) using a Wilcoxon signed-rank test (Z = 425.0, p < .001) as both distributions were not normally distributed according to the Shaprio-Wilks test of normality. Furthermore, it was found that the first (Z = 313.0, p < .001) and second rumbles (Z = 2237.0, p < .001) were performed significantly more quickly compared with the duration of the single rumble gesture.

Figure 7 (e) shows the average time between rumbles for the consecutive rumbling condition. In contrast to the double rumble, it is observed that the time difference between rumbles when participants were asked to perform for 5 seconds was significantly longer (Median = 662 ms), and more comparable to the duration of a single rumble. A Wilcoxon signed-rank test showed there was no statistically significant difference between the single rumble and those performed during the repetition task.

### Classifier performance evaluation:

To assess the performance of different classifiers under ideal conditions, the labeled start and end times of the different rumbles and time-constrained noise activities were used. For the continuous activities, random sub-samples were taken from the recording (1 - 3 seconds). To avoid over-fitting the noise class, random selections were taken evenly per class from all activities. For each classifier candidate, a 5-fold nested cross-validation with grid search for hyper-parameter optimization was performed. Both silent and music conditions were included in the training and test sets.

The best classifier was the XGBoost model which achieved 95% overall accuracy. The optimal hyper-parameters to achieve this were learning rate (0.08), maximum depth (3), and number of estimators (640). Table 1 shows the performance metrics of the different classifiers. The random forest classifier achieves similarly good performance overall, but it was found that it did not perform as well on the single rumble class.

**Table 1: Performance metrics comparison of different classifiers. XGBoost yielded the best overall performance for the individual rumbling activities.**

| **Classifier** | **Pre.** | **Rec.** | **F1** | **Acc.** |
|---|---|---|---|---|
| Dummy | 0.28 | 0.28 | 0.28 | 0.28 |
| RBF Kernel SVM | 0.81 | 0.79 | 0.80 | 0.79 |
| kNN | 0.82 | 0.81 | 0.81 | 0.81 |
| Random Forest | 0.95 | 0.95 | 0.95 | 0.95 |
| **XGBoost** | **0.95** | **0.95** | **0.95** | **0.95** |

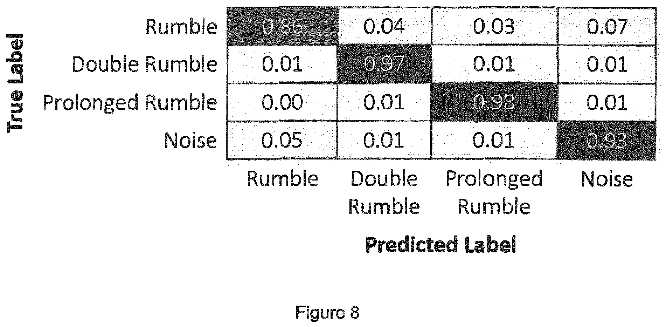

Figure 8 shows the confusion matrix of the best classifier. Prolonged and double rumble achieve the best results. The main reason for the confusion between rumbles and noise classes is that rumbles are much less significant in their structure and therefore might be confused easier with short noise samples. Likely, with additional sensors (e.g. IMU or microphone) the noise classes could be discriminated with higher accuracy from the rumble class and vice-versa.

### Leave one subject out-Validation:

There may be variations in the data for the different rumble gestures between participants. The variability could either be temporal (e.g. completion time of rumbles) and/or due to differences in the intensity of the ear rumble (*i.e.,* peak amplitude of the rumbles). Therefore, the best classifier was trained in a leave-one-subject-out cross-validation setting. Overall the classifier achieved 93% overall accuracy (2% decrease), which suggests that the proposed selection of features generalizes well across participants.

### Discussion:

It has been demonstrated how in-ear barometry can be used to detect contraction of the tensor tympani. The hold rumble gesture was particularly difficult to identify ground truth labels for, and this inherently has ramifications for the development of a classification pipeline because there are fewer samples for training and validation for the hold rumble. However, participants found contraction of the tensor tympani easy to perform on-demand and comfortable across the different ear rumble gestures.

Ear rumbling can be detected in silence or with music playing, however one participant mentioned that it was harder to focus on the execution of rumbles because the music made the rumbling sound harder to hear which affected the feedback loop. This is something that was potentially observed in the previous online questionnaire with the deaf participant and the participant with mild tinnitus, as feedback of the gesture is an important part of the interaction to notify that their interaction has been successfully registered. However, it also important to note that in this context there was no action associated with the ear rumbling gestures, and feedback can be provided either indirectly through the response of the system (e.g. changing the song or answering the phone), or directly in response to detection of the ear rumbling itself (e.g. play a sound to indicate rumbling).

There are a number of interesting insights to be gained from the analysis of rumbling characteristics in the above subsubsection "Analysis of ear rumble gestures". The acquisition time of an input technique refers to the time required to acquire the input device so that it is ready for use (e.g. unsheathing a pen), and the homing time refers to the time required to return to a "home" position (e.g. making contact with a finger for touch screen interaction). Ear rumbling through contraction of the tensor tympani does not introduce any acquisition time, nor any homing time. During the data collection participants were presented with a countdown timer prior to performing the ear rumble gestures, therefore it can be seen how quickly users are able to respond to the predicted stimulus.

No significant difference was observed for the start times between performing a single or double rumble, however it is noted that it takes approximately 308 ms to begin the ear rumble. This may be due to the fact that voluntary contraction of the tensor tympani is rarely performed as it serves little purpose. Comparing the time to start a rumble gesture, it is noted how it is nearly 100 ms shorter than to home in on a device (400 ms) according to the keystroke-level model (KLM). Based on the duration of a single ear rumble, it is noted how it is similar to typing random letters (500 ms) or complex codes (750 ms) according to KLM.

The present insights into repetitive rumbling indicate that contraction of the tensor tympani could be suitable for more complex interactions than the three basic gestures explored herein, and opens up the opportunity to use ear rumbling with switch scanning interfaces, rhythmic patterns, or beat synchronization. The present results provide insights into what kind of tempo one could use for these interactions to optimize throughput of the technique, and participants reported that this was generally easy to perform on-demand and comfortable. There is also scope to incorporate ear rumbling interaction with existing techniques, so that a wider vocabulary is available for users when interactions beyond simple binary choices are required.

### Example 4:

### Usability Evaluation

A study was performed to explore the performance and usability of EarRumble as a hands- and eyes-free input technique. Using a real-time implementation of the pipeline featured in Example 1, subsection "Recognition pipeline" the exploration was grounded in three manual, dual task application scenarios that one might face when using earables in everyday life. The goals of the study were to test how well the pipeline worked, and to gather feedback from users when using EarRumble for interaction.

### Participants and apparatus:

Eight participants (7 male, 1 female, Age: M = 24.5 SD = 2.7) from the previous data collection study in Example 3 were invited. Participants were seated in a regular office chair in front of a desktop PC. The pressure sensors in the earphones were connected via USB to a desktop computer which was running the EarRumble detection pipeline software in Python. Participants used the custom-built EarRumble earphones described in Example 1, and a separate pair of HolyHigh in-ear earables which features a mechanical click button on the outside of both earphones. Participants were instructed to use either earphone for the click, depending on their handedness preference.

### Design:

Three rumble gestures (single rumble, double rumble, and hold rumble) using the EarRumble technique were compared with a simple button click on a pair of smart earphones using three analogous gestures (click, double click, hold). A button click was chosen instead of a tap gesture because the button allows for the accurate detection of hold gestures. Three different use cases using two applications were chosen for the evaluation. These were chosen to evaluate the techniques in the context in which they may be used in real-life.

### Incoming call:

The first application scenario featured an incoming call, whereby a ringtone plays in the earphones and the user can either accept (rumble/click), reject (double rumble/click), or mute (hold rumble/button) the incoming phone call. A use case in which the user's attention is on a manual task was mimicked. Participants were given a primary task of typing a piece of text on the desktop PC whilst playing music in the background through the earphones. The phone would then ring, and the user would be tasked with either accepting, rejecting, or muting the phone call, before carrying on with the typing task.

### Audio player:

The second and third application scenarios feature an audio player in which the user can play and pause (rumble/click), skip to the next track (double rumble/click), or go back to the previous track (hold rumble/button). The second scenario consists of an audio transcription task whereby participants are tasked with transcribing sentences being read aloud through the earphones. After each sentence the participant is required to pause the audio, write the sentence down, and resume playback to hear the next sentence. The third scenario consisted of a music playlist in which participants were required to skip forwards or backwards in order to find specific songs.

### Procedure:

Participants began the study by completing demographic information and signing a consent form. They were then given one of the application tasks to practice with, using both input techniques. The order in which the applications were presented to participants were counterbalanced, as was the order of the input techniques.

For the incoming call scenario, participants completed the task in three blocks. For each block, the participant had to accept/reject/mute all incoming calls. Only one action was chosen per block to reduce the burden of memorizing which action to take. In each block, the participant received four phone calls at intervals of 20 seconds. This was chosen to give the participant time to resume typing, and long enough to reduce the chance of precisely predicting when a call would occur. For the audio player scenario, participants were given four sentences to transcribe, resulting in eight rumbles/clicks in order to pause and resume playback. The song play list consisted of five songs, and participants were tasked with finding the songs which involved skipping 3x forward, 2x back, 1x forward, and 2x back - resulting in four gestures each of the double rumble/click and hold rumble/click.

For the incoming call task, the response time of participants was measured from when the call was triggered to the corresponding action. The time taken to return to the typing task, defined as the first keystroke after the incoming call had been actioned (e.g. rejected) was also measured. Participants were asked to report any errors during the interactions arising from (a) incorrect detection of gestures (e.g. rumbles/clicks which aren't detected, or which should have been), (b) incorrect classification of gestures (e.g. detection of a single rumble/click instead of a hold rumble/click), and (c) user error (i.e., performing the wrong gesture). Participants completed the NASA Task load index (NASA TLX) as known in the art and were asked what they liked and disliked about each input technique for both of the applications. After both techniques had been performed for an application, participants were asked which they preferred using for the specific application, and at the end of the study they were asked which one they preferred overall.

### Results:

When asked about their overall preferences, six participants preferred the EarRumble technique, and two preferred the button click. The EarRumble technique was favored because it required less effort (P2, P4, P6, P8), and was more comfortable (P5). P3 specifically highlighted the single ear rumble gesture was their preferred technique. The button click was preferred because it was more robust (P1), and P7 noted that they would have preferred the earphones if it was tap input rather than button click because the EarRumble was lacking immediate feedback. Issues with reliably detecting the hold rumble were discovered - only 56% of prolonged rumbles were detected correctly compared with 91% of single rumbles and 94% of double rumbles throughout all tasks and across participants. A Friedman test was run on the responses to the NASA TLX but no significant differences between the input technique and task conditions were found. In the following the feedback from the two application scenarios is discussed.

### Phone call task:

For the phone call task, seven participants highlighted the advantage of not having to take their hands off the keyboard, and ability to continue typing immediately. P6 perceived that it felt much quicker than the button to interact. Excluding erroneous detections, the mean time between the phone call and detection of gesture was 3.40 s for EarRumble, and 3.34 s for the button click. The mean time between detection and the first keystroke during the call task was 1.09 s for EarRumble and 1.51 s for the button click. Statistical tests due to the different numbers of successful detections and low participant numbers were not run. Interestingly, P1 also noted how it was nice not to have to use voice - alluding to the social acceptability of the technique. However, six participants struggled using the hold rumble for interaction. P1, P5, and P7 reported that the hold rumble required additional concentration and was more uncomfortable to perform. P4 also commented on the latency of the rumble detection, and both P1 and P7 noted the lack of immediate feedback from the EarRumble techniques.

The participants also saw advantages of using the button for input, because it was clear how it worked and have used it before (P1, P7), provided immediate haptic feedback (P1, P4, P8), and worked reliably (P1, P3) with low latency (P1). However, 3 participants highlighted a disadvantage of having to take their hands off the keyboard to interact (P1, P2, P4) which they felt broke their workflow. Also, five participants felt the physical click button was hard to press and hurt the ear canal (P2, P3, P5, P6, P7). Two preferred the button click because of the immediate feedback (P1) and because it was more robust (P7).

Six out of the eight participants preferred the EarRumble technique for the phone call task. Participants preferred EarRumble because it required no extra movement (P2, P6), less time (P5), and allowed them to continue typing (P3, P8). P4 preferred the technique because "it felt magical" and "almost felt telepathic".

### Audio player task:

Participants gave similar feedback for the audio player tasks. The EarRumble technique was perceived to be faster because it does not require the use of the hands (P1, P3, P4, P8), and was low effort (P5). P6 described the technique as "much more practical" than the button click. Interestingly, two participants described the interactions with the audio player using EarRumble as "fun" (P7, P8). The disadvantages cited once again referred to the hold rumble (P1, P2, P5, P6, P7, P8), and higher latency of rumbling detection (P3, P4, P5).

Feedback was similar as well for the button click with the audio player, with it being described as a known technique (P5), very robust (P1), and immediate haptic feedback was an advantage (P1, P4, P7, P8). Participants described it as "annoying" to take the hands off the keyboard when pausing the text to write (P1, P3, P4), and once again they highlighted that the click hurts the ear canal (P2, P3, P5, P6, P7, P8).

No participants changed their preference between phone call task and audio player task. Participants preferred the EarRumble technique because it was low effort (P3, P4, P8), and because it does not require the use of the hands (P6). P2 said they preferred EarRumble because they think it would be "perfect when listening to music in bed". P5 reported that they preferred EarRumble because the button click was uncomfortable to use, and the remaining participants preferred the button because it was more robust (P1, P7).

### Discussion:

This usability evaluation highlights the low-effort, hands-free nature of the EarRumble technique, which was the main motivation of adopting ear rumbling for interaction. Scenarios in which participants could imagine using the EarRumble technique included during focused work (P1, P2, P4), when hands are occupied (P2, P5, P8), for secretive input (P2, P3), to interact without any noise, e.g. speech (P3), or for music or calls (P6). However, as expected from the results in section 5, the pressure sensing technique did not always reliably detect the hold rumble gestures. It is also noted how the latency of the pipeline was an issue for some participants, which with further optimization could be further reduced allowing for quicker selection times.

## Claims

1. A system for sensing a subject's ear canal barometric pressure, comprising:
at least one sensor device comprising an in-ear plug for arrangement in the subject's ear canal, the sensor device being configured to provide sensor information comprising a barometric pressure level enclosed by the in-ear plug and the subject's ear canal;
an evaluation device coupled to the at least one sensor device for evaluating the sensor information provided by the sensor device, the evaluation device being configured to determine a time course of the barometric pressure and,
based on the time course of the barometric pressure, to determine a contraction of the tensor tympani muscle of the subject; and
an interaction device coupled to the evaluation device, the interaction device being configured to provide means of explicit input to at least one computing device based on the determined contraction of the tensor tympani muscle of the subject.

2. The system according to claim 1, comprising two sensor devices for arrangement in each of the subject's ear canals, respectively.

3. The system according to claim 1 or claim 2, wherein the evaluation device is configured to determine the time course of the barometric pressure in one or, in case two sensor devices are present, in both of the subject's ear canals.

4. The system according to any one of claims 1 to 3, wherein the evaluation device is configured to determine, based on the time course of the barometric pressure in one or both of the subject's ear canals,
(i) a singular tensor tympani contraction event, wherein a negative pressure peak below a threshold of the baseline pressure reading after contraction is followed by a positive peak above a threshold of the baseline pressure reading after relaxation within a defined time threshold;
(ii) a repeated tensor tympani contraction event, wherein a singular tensor tympani contraction event as defined in (i) occurs two or more times in a row; and/or
(iii) a prolonged tensor tympani contraction event over a defined time threshold, wherein a negative pressure peak in the baseline pressure below a threshold after contraction is followed by a neutral pressure similar to the baseline pressure reading within a threshold while holding the muscle contracted and then a positive peak above a threshold of the baseline pressure after relaxation.

5. The system according to claim 4, wherein the time threshold can be freely defined.

6. The system according to any one of claims 1 to 5, wherein the evaluation device is configured to determine, based on the time course of the barometric pressure in one or both of the subject's ear canals, one or more or all features of said time course, selected from the group consisting of a positive pressure peak above a threshold of the baseline pressure, a negative pressure peak below a threshold of baseline pressure, the rising or falling time of a respective positive or negative pressure peak, the slope of a respective positive or negative pressure peak, the correlation of the barometric pressure at a given point in time as determined in both of the subject's ear canals, the number of respective positive and/or negative pressure peaks, the mean distance between respective positive and/or negative pressure peaks, the absolute difference of the first two of respective positive and/or negative pressure peaks, the minimum and/or maximum values of respective positive and/or negative pressure peaks, the location of respective positive and/or negative pressure peaks over time, the absolute difference between respective positive and/or negative pressure peaks, the ratio of maximum and/or minimum of respective positive and/or negative pressure peaks to the mean pressure level, the minimum slope and/or maximum slope and/or intercept of respective positive and/or negative pressure peaks over a five sample rolling window, the sum of absolute differences (SAD) of the pressure signal, the auto-correlation of the pressure signal, the variance of quantile changes of the pressure signal, the spectral welch density of the pressure signal, the continuous wavelet transform of the Ricker wavelet of the pressure signal, and the FFT coefficients of the pressure signal.

7. The system according to claim 6, wherein a tensor tympani contraction event is calculated by automated means from said one or more features of said time course by a machine learning classifier.

8. The system according to claim 7, wherein the machine learning classifier is selected from the group consisting of a decision tree, a support vector machine, a random forest classifier, and a neural network.

9. The system according to any one of claims 1 to 8, wherein the interaction device is configured to use the determined contraction of the tensor tympani muscle of the subject to provide means of explicit input to one or more computing device(s) to effect control of said computing device(s).

10. The system according to any one of claims 1 to 9, wherein the in-ear plug is selected from the group consisting of a soft silicone tip, a molded earpiece based on the subject's ear canal, and an expanding foam tip.

11. The system according to any one of claims 1 to 10, wherein said system is integrated in an earphone device.

12. A method of providing explicit input to at least one computing device, comprising the steps of
(i) detecting a contraction of the tensor tympani muscle of a subject, and
(ii) using a detected contraction event as the input to the at least one computing device.

13. Use of the contraction of the tensor tympani muscle of a subject as explicit input to at least one computing device.

14. The method according to claim 12 or the use according to claim 13, wherein the contraction of the tensor tympani muscle is a singular tensor tympani contraction event, a repeated tensor tympani contraction event, or a prolonged tensor tympani contraction event over a defined time threshold.

15. The method according to claim 12 or claim 14, or the use according to claim 13 or claim 14, wherein the contraction of the tensor tympani muscle of the subject is detected using the system according to any one of claims 1 to 11.
